Europäisches Patentamt

European Patent Office

Office européen des brevets

(19)

(11) EP 0 960 875 B1

(12) **EUROPÄISCHE PATENTSCHRIFT**

(45) Veröffentlichungstag und Bekanntmachung des Hinweises auf die Patenterteilung:
**29.08.2001  Patentblatt 2001/35**

(51) Int Cl.[7]: **C07C 53/08**, C07C 51/25

(21) Anmeldenummer: **99108644.8**

(22) Anmeldetag: **12.05.1999**

(54) **Verfahren zur Herstellung von gesättigten Carbonsäuren mit 1 bis 4 C-Atomen**

Process for the preparation of saturated carboxylic acids with 1-4 C-atoms

Procédé pour la préparation d'acides carboxyliques saturés avec 1 à 4 atomes de carbone

(84) Benannte Vertragsstaaten:
**AT BE CH DE ES FI FR GB IT LI NL SE**

(30) Priorität: **22.05.1998  DE 19823088**
**10.03.1999  DE 19910628**

(43) Veröffentlichungstag der Anmeldung:
**01.12.1999  Patentblatt 1999/48**

(73) Patentinhaber: **Consortium für elektrochemische Industrie GmbH**
**81379 München (DE)**

(72) Erfinder:
• **Rüdinger, Christoph, Dr.**
**81497 München (DE)**
• **Eberle, Hans-Jürgen, Dr.**
**81477 München (DE)**

• **Bogner, Ragnar**
**81671 München (DE)**
• **Kohlmann, Wolfgang**
**81739 München (DE)**

(74) Vertreter: **Schuderer, Michael, Dr. et al**
**Wacker-Chemie GmbH**
**Zentralabteilung Patente**
**Marken und Lizenzen**
**Hanns-Seidel-Platz 4**
**81737 München (DE)**

(56) Entgegenhaltungen:
EP-A- 0 546 677     DE-A- 2 149 752
DE-B- 1 279 011     GB-A- 1 010 068
US-A- 4 158 740

**Beschreibung**

Gegenstand der Erfindung ist ein Verfahren zur Herstellung von gesättigten Carbonsäuren mit 1 bis 4 C-Atomen, sowie eine Vorrichtung zur Durchführung des Verfahrens.

Es ist bekannt, daß Essigsäure durch Gasphasenoxidation von $C_4$-Kohlenwasserstoffen in Gegenwart eines Katalysators hergestellt werden kann. Die meisten Beschreibungen sehen vor, das Reaktionsgasgemisch einmal über den Katalysator zu leiten, die gebildete Essigsäure durch Kondensation oder Auswaschen abzutrennen und das verbleibende Gas zu verwerfen. In der US-A 3,917,682 wird beispielsweise ein Vorgehen beschrieben, bei dem Essigsäure durch Butenoxidation in Gegenwart eines Ti/V-Katalysators mit hohem Rutilanteil gewonnen wird, wobei die Essigsäure durch Partialkondensation des Reaktionsgemisches isoliert wird, der verbleibende Rest des Reaktionsgases wird nicht zurückgeführt. Derartige Verfahren müssen bereits bei einmaligem Reaktordurchgang einen hohen Butenumsatz erreichen, was nur mit geringen Ausbeuten oder geringen Raum-Zeit-Leistungen gelingt. Auf dieser Verfahrensbasis konnte deshalb bisher noch kein wirtschaftlich befriedigendes Verfahren gefunden werden.

Aus der US-A 4,146,734 ist bekannt, die Gasphasenoxidation von Buten zu Essigsäure in Gegenwart eines Lanthaniden-Verbindungen enthaltenden Katalysators durchzuführen. Ein Verfahren zur Gewinnung der Essigsäure und weiterer während der Gasphasenoxidation gebildeter Wertstoffe wird nicht angegeben. Die DE-A 2149752 und die DE-A 1279011 beschreiben Verfahren zur katalytischen Gasphasenoxidation von Buten zu Essigsäure in Gegenwart von speziellen Katalysatoren. Nachteilig bei dieser Verfahrensweise ist, daß bei der dort angegebenen Rückführung des nicht kondensierbaren Anteils des Reaktionsgases der Anteil der als Wertstoff anfallenden Ameisensäure zerfällt. In der DE-A 1921503 wird auf die Möglichkeit hingewiesen bei der Essigsäureherstellung mittels katalytischer Gasphasenoxidation von Buten den nicht reagierten Anteil des Reaktionsgemisches in den Reaktor zurückzuführen, es wird aber ausdrücklich auf die Unwirtschaftlichkeit eines Kreisgasverfahrens verwiesen.

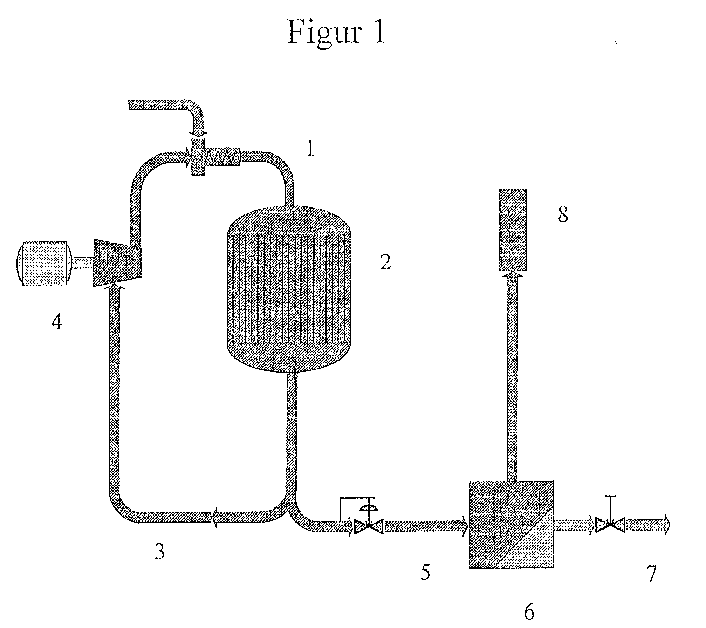

Das von den Chemischen Werken Hüls zum Pilotmaßstab entwickelte, und in verschiedenen Publikationen (R.P. Lowry, A. Aguilo, Hydrocarbon Processing, 10, (1974), 103; PEP Report No. 37A (1973)) beschriebene Verfahren sieht die direkte, unbehandelte Rückführung von 4/5 des den Reaktor verlassenden Gasgemisches vor (Figur 1). Bei dieser Ausführungsform wird das Reaktionsprodukt teilweise ohne Abtrennung der Säuren im Kreis geführt und nur ein Teil zur Isolierung der Essigsäure abgetrennt. Bei diesem Verfahren kommt es zu einer deutlichen Anreicherung von organischen Säuren im Reaktionsgas, wodurch sowohl Essigsäure als auch Ameisensäure nur in nicht befriedigender Ausbeute erhalten werden.

Es bestand daher die Aufgabe ein Verfahren zur Herstellung von gesättigten Carbonsäuren mit 1 bis 4 C-Atomen, insbesondere Essigsäure, durch Gasphasenoxidation von gesättigten und/oder ungesättigten $C_4$-Kohlenwasserstoffen zu finden, welches hohe Saureausbeuten liefert und bei dem die Nebenprodukte als Wertstoffe anfallen.

Es wurde überraschend gefunden, daß sich die Herstellung von gesättigten Carbonsäuren mit 1 bis 4 C-Atomen durch Gasphasenoxidation von gesättigten und/oder ungesättigten $C_4$-Kohlenwasserstoffen mit besonders hohen Ausbeuten durchführen läßt, wenn im Gegensatz zu den obengenannten Verfahren ein von Sauren überwiegend befreiter Teilstrom des Reaktionsausgangsgasgemisches an den Reaktoreingang zurückgeführt wird.

Gegenstand der Erfindung ist ein Verfahren zur Herstellung von gesättigten Carbonsäuren mit 1 bis 4 C-Atomen durch Gasphasenoxidation bei einer Reaktionstemperatur von 100°C bis 400°C und bei Drücken von $1,2 \cdot 10^5$ Pa bis $51 \cdot 10^5$ Pa, in Gegenwart von gesättigten und/oder ungesättigten $C_4$-Kohlenwasserstoffen, einem sauerstoffhaltigen Gas und Wasserdampf in Anwesenheit von zumindest einem Katalysator wobei das Reaktionsausgangsgas in einem Reaktionsgaskreislauf zum Teil zurückgeführt wird, dadurch gekennzeichnet, daß der Reaktionsgaskreislauf so ausgeführt wird, daß dem Reaktionsausgangsgas ein Teil der bei der Gasphasenoxidation entstandenen organischen Säuren entzogen wird, sodaß der Säureanteil im zurückgeführten Anteil des Reaktionsausgangsgases zwischen 0,01 und 6,0 Vol.-% liegt.

Bei den gesättigten oder ungesättigten Kohlenwasserstoffen mit vier Kohlenstoffatomen handelt es sich um Verbindungen aus der Gruppe umfassend n-Butan, i-Butan, t-Butan, 1-Buten, cis-2-Buten, trans-2-Buten, Isobuten, 1,3-Butadien. Bevorzugt sind n-Butan und die Buten-Isomere 1-Buten, trans-2-Buten und cis-2-Buten sowie Gemische, die diese Verbindungen in hohen Anteilen enthalten. Bei dem erfindungsgemäßen Verfahren kann die $C_4$-Kohlenwasserstofffraktion auch lineare und/oder verzweigte und/oder cyclische Kohlenwasserstoffe mit mehr oder weniger als vier Kohlenstoffatomen wie beispielsweise Methan, Ethan, Ethen, Propen, Propan, Pentane, Pentene, Pentadiene, Cyclopentan, Cyclopenten, Cyclopentadien, Methylcyclopentan enthalten. Ebenso können Alkohole, Aldehyde, Ether, Ketone und Ester mit 1 bis 8 Kohlenstoffatomen anwesend sein. Bevorzugt werden billige Rohstoffgemische aus der Petrochemie wie "$C_4$-Schnitt" (überwiegender Anteil an Butadien und i-Buten), "Raffinat 1" (überwiegender Anteil an i-Buten und n-Butenen) und "Raffinat 2" (überwiegender Anteil an Butanen, 1-Buten und 2-Butenen) als Ausgangsmaterial oder Mischungen die solche Kohlenwasserstoffe enthalten. Diese können gegebenenfalls nach einer Vorbehandlung z.B. einer Reinigung bzw. Hydrierung verwendet werden.

Die Reaktionstemperatur der Gasphasenoxidation beträgt im allgemeinen 100°C bis 400°C, vorzugsweise 150°C bis 250°C, besonders bevorzugt 180°C bis 230°C. Die Reaktion wird im allgemeinen bei Drücken zwischen

1,2·10$^5$ Pa bis 51·10$^5$ Pa, bevorzugt zwischen 4·10$^5$ Pa und 31·10$^5$ Pa, besonders bevorzugt zwischen 9·10$^5$ und 17·10$^5$ Pa durchgeführt.

Als sauerstoffhaltiges Gas kann Luft, mit Sauerstoff angereicherte Luft und bevorzugt reiner Sauerstoff verwendet werden.

Bei dem erfindungsgemäßen Verfahren kann auch ein Inertgas wie beispielweise Stickstoff, anwesend sein.

Der Volumenanteil von Wasserdampf, sauerstoffhaltigem Gas, $C_4$-Kohlenwasserstoffen, und Inertgasen des dem Reaktor zugeführten Reaktoreingangsgases beträgt im allgemeinen 5 bis 80 Vol.-%, vorzugsweise 5 bis 40 Vol.-%, besonders bevorzugt 5 bis 30 Vol.-% Wasserdampf.

Der Anteil an Buten, welches allein oder im Gemisch mit weiteren $C_4$-Kohlenwasserstoffen als Edukt vorliegen kann, beträgt 1 bis 5 Vol.-% ,vorzugsweise 1,5 bis 3 Vol.-%. Der Anteil an Butan , welches ebenfalls allein oder im Gemisch mit weiteren $C_4$-Kohlenwasserstoffen als Edukt vorliegen kann, beträgt 5 bis 80 Vol.-%, vorzugsweise 5 bis 60 Vol.-%, besonders bevorzugt 10 bis 50 Vol.-%.

Der Sauerstoffgehalt im Reaktoreingangsgas beträgt 1 bis 35 Vol.-%, vorzugsweise 2 bis 20 Vol.-%, besonders bevorzugt 3 bis 12 Vol.-%.

Gegebenenfalls kann ein Inertgasanteil von 0 bis 25 Vol.-% zugeführt werden. Der Anteil an Kohlenoxiden und weiteren Reaktionsnebenprodukten im Reaktoreingangsgas hängt von der Reaktionsführung und Säureabtrennung ab und beträgt im allgemeinen von 10 bis 80 Vol.-%, bevorzugt von 15 bis 65 Vol.-%. Die Anteile in Vol.-% der einzelnen Bestandteile des Reaktoreingangsgases addieren sich dabei jeweils auf 100 Vol.-%.

Als Katalysatoren für das erfindungsgemäße Verfahren eignen sich alle Katalysatoren die allgemein für die partielle Oxidation von gesättigten und/oder ungesättigten $C_4$-Kohlenwasserstoffen zu Essigsäure beschrieben sind. Bevorzugt sind Mischoxidkatalysatoren die Vanadiumoxide enthalten, besonders bevorzugt sind Schalenkatalysatoren wie sie in der DE-A 19649426 beschrieben sind. Die DE-A 19649426, deren diesbezügliche Offenbarung Teil dieser Anmeldung sein soll, wird hiermit unter Bezugnahme eingeschlossen (incorporated by reference). Dabei handelt es sich um einen Schalenkatalysator bestehend aus einem inerten unporösen Trägerkörper und einer auf die äußere Oberfläche des Trägerkörpers aufgebrachten katalytisch aktiven Mischoxidmasse, welche a) ein oder mehrere Oxide aus der Gruppe Titandioxid, Zirkoniumdioxid, Zinndioxid, Aluminiumoxid und b) 0,1 bis 1,5 Gew.-%, bezogen auf das Gewicht der Komponente a) und pro m$^2$/g spezifischer Oberfläche der Komponente a), Vanadiumpentoxid enthält.

Als zusätzliche Komponente a) können noch ein oder mehrere Oxide aus der Gruppe Bor, Silicium, Hafnium, Niob, Wolfram, Lanthan und Cer enthalten sein. Bei der Dotierung der Komponente a) mit den genannten Oxiden sind diese im allgemeinen in einer Menge von 1 bis 30 Gew.-%, bezogen auf das Gesamtgewicht der Komponente a), enthalten.

Bei der Komponente b) kann gegebenenfalls ein Teil des Vanadiumpentoxids, vorzugsweise 10 bis 90 Gew.-%, durch ein oder mehrere Oxide von Molybdän, Chrom und Antimon ersetzt werden. Gegebenenfalls können als zusätzliche Komponente b) noch ein oder mehrere Oxide von Alkalimetallen, Elementen der 5. und 6. Hauptgruppe des Periodensystems der Elemente (PSE) und der Übergangsmetalle enthalten sein. Im allgemeinen beträgt die Menge dieser Dotierstoffe 0,005 bis 15 Gew.-%, berechnet als Oxide und bezogen auf das Gesamtgewicht der Komponente b).

Bevorzugt werden Zusammensetzungen mit hoher Oberfläche der Komponente a) von 40 bis 300 m$^2$/g, wobei gegebenenfalls noch Zinn- Niob- oder Wolframoxid enthalten sein können, und mit einer Komponente b), welche mit Mo, und/oder Cr, und/oder Sb und/oder Au dotiert ist. Die katalytisch aktive Mischoxidmasse kann gegebenenfalls noch 10 bis 50 Gew.-%, bezogen auf das Gesamtgewicht der katalytisch aktiven Mischoxidmasse, inerte Verdünnungsmittel wie Siliciumdioxid, Siliciumcarbid, Graphit enthalten.

Die katalytisch aktive Mischoxidmasse ist in einem Anteil von 1 bis 40 Gew.-%, vorzugsweise 5 bis 25 Gew.-%, jeweils bezogen auf das Gesamtgewicht aus Trägerkörper und aktiver Masse, als Schale auf die äußere Oberfläche des Trägerkörpers aufgebracht. Die Schichtdicke beträgt im allgemeinen 10 bis 2000 µm, vorzugsweise 100 bis 1000 µm. Der Schalenkatalysator kann auch mehrere, sich in der Zusammensetzung unterscheidende, Schichten enthalten. Es können auch ein oder mehrere Bestandteile der Aktivkomponenten a) und b) in unterschiedlicher Konzentration in den einzelnen Schichten enthalten sein.

Geeignete Materialien für den inerten, unporösen Trägerkörper sind alle unter den Betriebsbedingungen der Gasphasenoxidation sich inert verhaltenden und über den Betriebszeitraum stabilen unporösen Materialien. Beispiele hierfür sind Steatit, Duranit, Siliciumcarbid, Magnesiumoxid, Siliciumoxid, Silikate, Aluminate, Metalle wie Edelstahl, sowie gegebenenfalls Mischungen aus diesen Stoffen. Bevorzugt wird keramisches Material wie Steatit. Die Formgestalt des inerten, unporösen Trägerkörpers ist beliebig. Beispiele für geeignete Formen sind Kugeln, Zylinder, Quader, Tori, Sättel, Spindeln, Wendeln. Ebenfalls als Träger geeignet sind geordnete Packungen wie Monolithe oder Kreuzkanalstrukturen. Bevorzugt sind Trägerformen mit möglichst hoher geometrischer Oberfläche pro Volumen, beispielsweise Ringe.

Die Abmessungen der Trägerkörper sind durch die Reaktoren zur Gasphasenoxidation vorgegeben. Im allgemeinen haben die Formkörper eine Länge bzw. einen Durchmesser von 2 bis 20 mm. Die Wanddicke, beispielsweise im Fall von Ringen oder Hohlzylindern, beträgt zweckmäßigerweise 0,1 bis 4 mm.

Bei dem erfindungsgemäßen Verfahren wird der Reaktionsgaskreislauf so ausgeführt, daß dem Reaktionsausgangsgas ein Teil der darin vorhandenen organischen Säuren, primär Ameisensäure und Essigsäure, entzogen wird, so daß der Partialdruck dieser Säuren am Reaktoreingang niedrig bleibt. Im allgemeinen wird der Säureanteil auf 0,01 bis 6 Vol.-%, bevorzugt 0,1 bis 3 Vol.-% besonders bevorzugt 0,2 bis 2 Vol.-% reduziert. Nicht umgesetzte $C_4$-Kohlenwasserstoffe und weiter zu Essigsäure umsetzbare Zwischenprodukte wie Acetaldehyd, Aceton, Methylethylketon, 2-Butanol verbleiben größtenteils im Kreisgas und werden zum Reaktoreingang zurückgeführt.

Man kann dabei so vorgehen, daß bei einem Teil des Reaktorausgangsgases, im allgemeinen 60 bis 99,8 Gew.-%, vorzugsweise 90 bis 99,5 Gew.-%, der Säureanteil bis zu dem obengenannten. Restsäuregehalt abgetrennt wird, und anschließend dieser Teil des Reaktorausgangsgases wieder in den Reaktor zurückgeführt wird. Der unbehandelte Teil des Reaktorausgangsgases wird verworfen und kann beispielsweise abgefackelt werden. Der Anteil an unbehandeltem Reaktorausgangsgas hängt davon ab, wieviel Kohlenoxid $CO_x$ gebildet worden sind, weil diese über diesen Zweigstrom abgeführt werden müssen. Sie können danach mittels Verbrennung entsorgt werden

Es kann auch so vorgegangen werden, daß dem Reaktorausgangsgas unmittelbar nach dem Austritt aus dem Reaktor der Säureanteil bis zu dem obengenannten Restanteil abgetrennt wird, und das so behandelte Reaktorausgangsgas ganz oder teilweise, vorzugsweise zu einem Anteil von 60 bis 99,8 Gew.-%, besonders bevorzugt von 90 bis 99,5 Gew.-% in den Reaktor zurückgeführt wird. Diese Ausführungsform ist besonders bevorzugt, da dabei die Zielprodukte, die Carbonsäuren, vorher weitgehend abgetrennt werden und nicht in die Verbrennung gehen.

Die Abtrennung der organischen Säuren kann mittels bekannter Methoden oder einer Kombination dieser Methoden erfolgen. Beispielsweise durch partielle Kondensation des Gasgemisches; durch Rektifikation, gegebenenfalls unter Zusatz von Hilfsstoffen (z.B. Extraktivrektifikation); durch Absorption der Säuren in einem geeigneten Lösungsmittel; mittels einer Membran; durch einen Festabsorber. Bevorzugt wird die Abtrennung der organischen Säuren mittels partieller Kondensation des Gasgemisches, beispielsweise in einem Kühler, und anschließender Aufteilung des verbleibenden Gasgemisches.

Der Anteil des rückgeführten Gasmassenstroms beträgt im allgemeinen zwischen dem 1-fachen und dem 100-fachen des frisch zugeführten Eduktmassenstroms, bevorzugt zwischen dem 5-fachen und 80-fachen, besonders bevorzugt zwischen dem 10- bis 40-fachen.

Die abgetrennte Rohsäure wird mit geeigneten üblichen Verfahren alleine oder in Kombination wie Flüssig-Flüssig-Extraktion, Extraktivrektifikation, Azeotroprektifikation und Rektifikation entwässert und gereinigt. Das erfindungsgemäße Verfahren eignet sich vorzugsweise zur Herstellung von Essigsäure und Ameisensäure, besonders bevorzugt Essigsäure. Ein wesentlicher Vorteil der erfindungsgemäßen Vorgehensweise besteht darin, daß bei der Herstellung von Essigsäure, die dabei entstehenden Nebenprodukte als Wertstoffe, vor allem in Form von Ameisensäure, anfallen. Demgegenüber wird bei den aus dem Stand der Technik bekannten Verfahren der intermediär entstehende Ameisensäureanteil zersetzt und es entstehen $CO_x$-Verbindungen, welche mittels Verbrennung entsorgt werden müssen.

Als Reaktor können Ausführungen verwendet werden, die sich für die Durchführung von Oxidationsreaktionen in der Gasphase eignen und in der Lage sind die hohe Reaktionswärme ohne übermäßige Erwärmung des Reaktionsgemisches abzuführen. Das erfindungsgemäße Verfahren kann kontinuierlich oder intermitierend durchgeführt werden, das heißt die Zuführung des Reaktoreingangsgemisches kann mit konstantem Feed oder mit zyklisch variierender Feedzusammensetzung erfolgen. Das Gasgemisch kann an dem Katalysator in einem Festbett, beispielsweise in einem Rohrbündelreaktor oder Hordenreaktor, oder in einem Fließ- bzw. Wirbelbett reagieren. Bevorzugt sind gekühlte Rohrbündelreaktoren mit festem Katalysatorbett. Besonders bevorzugt sind Ausführungen mit zu Rohrbündel angeordneten Einzelrohren mit einem Rohrinnendurchmesser von 10 mm bis 50 mm und einer Rohrlänge von 1 m bis 6 m. Die Strömungsgeschwindigkeit (bezogen auf das ungefüllte Rohr) in den Reaktionsrohren beträgt im allgemeinen zwischen 0,1 m/s und 10 m/s, bevorzugt zwischen 0,3 m/s und 5 m/s, besonders bevorzugt 0,5 bis 3 m/s.

Die Reaktionsrohre können mit Katalysator unterschiedlicher Zusammensetzung, Gestalt und Dimension gefüllt sein. Die Füllung kann in axialer Richtung homogen oder zonenweise variabel in die Reaktionsrohre eingebracht sein. Jede Zone kann statistisch verdünnten oder gemischten Katalysator enthalten.

Figurenbeschreibung:

Figur 1 beschreibt eine Anlage nach dem Stand der Technik. Hierbei wird über die Zufuhr (1) das Reaktionseingangsgas in einen Reaktor (2) zugeführt. Ein Teil des den Reaktor verlassenden Gasgemisches geht durch Rohr (5) zu der Trennvorrichtung (6), beispielsweise einem Kühler mit nachgeschaltetem Phasentrenner, wobei die Säure durch Teilkondensation gewonnen über Leitung (7) abgelassen wird und der gasförmige Restanteil der Verbrennung (8) zugeführt wird. Der andere Teil des Reaktorausgangsgases wird über Rohr (3), ohne Säureabtrennung, mittels einem Gasfördermittel, beispielsweise einem Gebläse, (4) zum Reaktoreingang zurückgeführt.

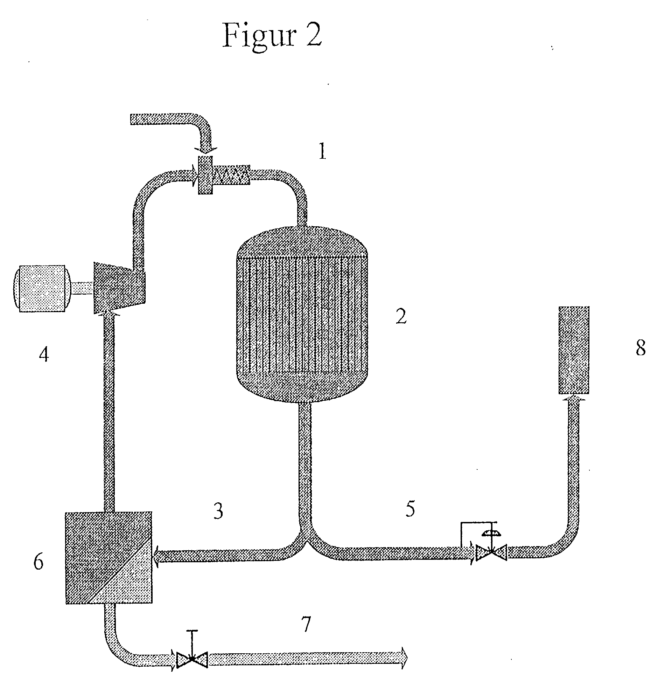

Figur 2 beschreibt eine Anlage, bei der die Säureabtrennung im Rückstrom erfolgt. Hierbei wird über die Zufuhr (1) das Reaktionseingangsgas in einen Reaktor (2) zugeführt. Aus diesem Reaktor geht der Großteil des Reaktorausgangsgases durch Rohr (3) zu der Trennvorrichtung (6), beispielsweise einem Kühler mit nachgeschaltetem Phasentrenner, wobei der Großteil der Säure durch Teilkondensation gewonnen wird und über Leitung (7) abgelassen wird. Der flüchtige Rest wird mittels eines Gasfördermittels (4), wobei es sich um ein Gebläse oder einen Kompressor handeln kann, zum Reaktoreingang rückgeführt. Durch Rohr (5) geht überschüssiges Gas, das bei der Druckreglung anfällt, zur Verbrennung (8).

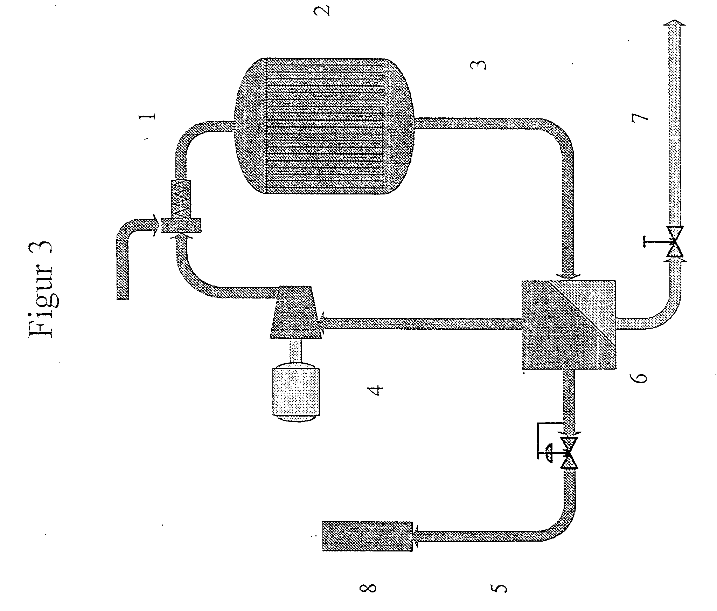

Figur 3 beschreibt eine Anlage bei der die Säureabtrennung vor der Gasstromteilung direkt am Reaktorausgang erfolgt. Hierbei wird über die Zufuhr (1) das Reaktionseingangsgas in einen Reaktor (2) zugeführt. Aus diesem Reaktor geht das Reaktorausgangsgas über Leitung (3) zu der Trennvorrichtung (6), beispielsweise einem Kühler mit nachgeschaltetem Phasentrenner, wobei der Großteil der Säure durch Teilkondensation gewonnen wird und über Leitung (7) abgelassen wird. Der flüchtige Rest wird mittels eines Gasfördermittels (4), wobei es sich um ein Gebläse oder einen Kompressor handeln kann, zum Reaktoreingang rückgeführt. Durch Rohr (5) geht überschüssiges Gas, das bei der Druckreglung anfällt, durch einen Druckregler zur Verbrennung (8).

Die folgenden Beispiele sollen die Erfindung näher erläutern:

Die Ausbeute [mol-%] wurde wie folgt berechnet:

mole Kohlenstoff in der Produktmasse / mole Kohlenstoff in der

eingesetzten Eduktmasse x 100

Reaktordimensionen:

In den Beispielen 1 bis 4 und den Vergleichsbeispielen:
Rohrreaktor mit 19 mm Rohrinnendurchmesser, 6 m Rohrlänge.
In Beispiel 5:
Rohrreaktor mit 25 mm Rohrinnendurchmesser, 6 m Rohrlänge.

Katalysatoren:

In Beispiel 1, 2 und den Vergleichsbeispielen 1 bis 3:
Schalenkatalysator mit aktiver Masse aus Oxiden von Titan, Vanadium, Molybdän und Antimon der empirischen Formel $Ti_aV_bMo_cSb_dO_e$ (a: 93; b: 7,4; c: 1; d: 2,8; e: 211), welcher in einem Anteil von 18 Gew.-% bezogen auf das Gewicht des Trägers auf Steatitringe der Dimension 7 mm Außendurchmesser, 4 mm Innendurchmesser, 4 mm Höhe aufgebracht ist.
In den Beispielen 3 und 4:
Schalenkatalysator mit aktiver Masse aus Oxiden von Titan, Vanadium, und Antimon der empirischen Formel $Ti_aV_bSb_dO_e$ (a: 11,8; b: 1; d: 1,18; e: 27,8), welcher in einem Anteil von 13,5 Gew.-% unter Zusatz von 1,5 Gew.-% Graphit, jeweils bezogen auf das Gewicht des Trägers, auf Steatitringe der Dimension 7 mm Außendurchmesser, 4 mm Innendurchmesser, 4 mm Höhe aufgebracht ist.
In Beispiel 5:
Schalenkatalysator mit aktiver Masse aus Oxiden von Titan, Vanadium, und Antimon der empirischen Formel $Ti_aV_bSb_dO_e$ (a: 11,8; b: 1; d: 1,18; e: 27,8), welcher in einem Anteil von 10,8 Gew.-% unter Zusatz von 1,2 Gew.-% Graphit, jeweils bezogen auf das Gewicht des Trägers, auf Steatitringe der Dimension 7 mm Außendurchmesser, 4 mm Innendurchmesser, 7 mm Höhe aufgebracht ist.

Beispiel 1:

(Kreisgasfahrweise mit Säureabtrennung nach Abbildung 2)

Ein Rohrreaktor mit 19 mm Innendurchmesser und 6 m Rohrlänge, welcher mit einer Heizeinrichtung ausgerüstet war, und mit dem oben angegebenen Katalysator befüllt war, wurde bei einem Druck von $7·10^5$ Pa und einer Temperatur von 190°C mit 600 g/h Wasser, 250 g/h Sauerstoff, 120 g/h 1-Buten beschickt.
Das aus dem Reaktor austretende Gasgemisch wurde getrennt. Ein Anteil von 60 g/h wurde einem Wäscher zugeführt und anschließend abgefackelt. Der verbliebene Anteil von 9000 g/h des Reaktorausgangsgases wurde bei 70°C in einem Kühler teilkondensiert, der flüssige Anteil mit der Essigsäure in einem Phasentrenner isoliert, und das Restgas zum Reaktoreingang zurückgeführt. Der Säureanteil im Kreisgas wurde so auf 1,2 Vol.-% reduziert.
Unter diesen Bedingungen wurde ein Butenumsatz von 99,5 % erreicht. Die Essigsäureausbeute betrug 71 mol-%, die Acetaldehydausbeute 0,5 mol-% und die Ameisensäureausbeute 9 mol-%. Die Raum-Zeit-Leistung erreichte 107 g Essigsäure pro Liter Katalysator und Stunde. Die Rohsäurekonzentration betrug 25 Gew.-%.

Beispiel 2:

(Kreisgasfahrweise mit Säureabtrennung nach Abbildung 2)

Es wurde analog Beispiel 1 vorgeganngen, wobei der Reaktor mit 600 g/h Wasser, 31 g/h Stickstoff, 250 g/h Sauerstoff und 120 g/h 1-Buten beschickt wurde. Das aus dem Reaktor austretende Gasgemisch wurde getrennt. Ein Anteil von 90 g/h wurde einem Wäscher zugeführt und anschließend abgefackelt. Der verbliebene Anteil von 9000 g/h des Reaktorausgangsgases wurde bei 70°C in einem Kühler teilkondensiert, der flüssige Anteil mit der Essigsäure in einem Phasentrenner isoliert, und das Restgas zum Reaktoreingang zurückgeführt. Der Säureanteil im Kreisgas wurde so auf 1,2 Vol.-% reduziert.

Unter diesen Bedingungen wurde ein Butenumsatz von 98 % erreicht. Die Essigsäureausbeute betrug 66 mol-%, die Acetaldehydausbeute 0,5 mol-% und die Ameisensäureausbeute 8,5 mol-%. Die Raum-Zeit-Leistung erreichte 99 g Essigsäure pro Liter Katalysator und Stunde. Die Rohsäurekonzentration betrug 23 Gew.-%.

Vergleichsbeispiel 1:

(Einfacher Reaktordurchgang)

Der Rohrreaktor aus Beispiel 1 wurde bei $5 \cdot 10^5$ Pa und 205°C mit 1600 g/h Wasser, 390 g/h Stickstoff, 130 g/h Sauerstoff und 113 g/h 1-Buten beschickt. Das Reaktionsausgangsgas wurde nicht im Kreis geführt, sondern nach dem Reaktordurchgang aufgetrennt. Unter diesen Bedingungen wurde ein Butenumsatz von 50 % erreicht. Die Essigsäureausbeute betrug 34 mol-%, die Acetaldehydausbeute 5 mol-% und die Ameisensäureausbeute 2 mol-%. Die Raum-Zeit-Leistung erreichte 48 g Essigsäure pro Liter Katalysator und Stunde. Die Rohsäurekonzentration betrug 5 Gew.-%.

Vergleichsbeispiel 2:

(Kreisgasfahrweise ohne Säureabtrennung)

Es wurde analog Beispiel 1 vorgegangen, wobei der Reaktor bei einem Druck von $4 \cdot 10^5$ Pa und einer Temperatur von 220°C mit 200 g/h Wasser, 143 g/h Sauerstoff und 130 g/h 1-Buten beschickt worden ist. 4700 g des Reaktorausgangsgases wurden ohne Saureabtrennung direkt zum Reaktoreingang zurückgeführt, 460 g/h des Reaktorausgangsgases wurden zur Produktgewinnung und Kohlenoxidabfuhr aus dem Kreisgassystem entnommen. Unter diesen Bedingungen wurde ein Butenumsatz von 60 %. erreicht. Die Essigsäureausbeute betrug 36 mol-%, die Acetaldehydausbeute 1,2 mol-% und die Ameisensäureausbeute 1,2 mol-%. Die Raum-Zeit-Leistung erreichte 60 g Essigsäure pro Liter Katalysator und Stunde. Die Rohsäurekonzentration betrug 30 Gew.-%.

Vergleichsbeispiel 3:

(Kreisgasfahrweise ohne Säureabtrennung)

Es wurde analog Vergleichsbeispiel 2 vorgegangen, wobei bei $7 \cdot 10^5$ Pa und 190°C 200 g/h Wasser, 490 g/h Stickstoff, 170 g/h Sauerstoff und 130 g/h 1-Buten dem Reaktor zugeführt wurden. 9100 g/h des Reaktorausgangsgases wurden ohne Säureabtrennung direkt zum Reaktoreingang zurückgeführt, 990 g/h des Reaktorausgangsgases wurden zur Produktgewinnung und Kohlenoxidabfuhr aus dem Kreisgassystem entnommen. Unter diesen Bedingungen wurde ein Butenumsatz von 46 % erreicht. Die Essigsäureausbeute betrug 29 mol-%, die Acetaldehydausbeute 3,2 mol-% und die Ameisensäureausbeute 2,3 mol-%. Die Raum-Zeit-Leistung erreichte 50 g Essigsäure pro Liter Katalysator und Stunde. Die Rohsäurekonzentration betrug 25 Gew.-%.

Beispiel 3:

(Kreisgasfahrweise mit Säureabtrennung nach Abbildung 2)

Es wurde analog Beispiel 1 vorgegangen, wobei der obengenannte Schalenkatalysator eingesetzt wurde, und 700 g/h Wasser, 310 g/h Sauerstoff, 120 g/h 1-Buten sowie 80 g/h n-Butan dem Reaktor bei $11 \cdot 10^5$ Pa und 200°C zugeführt wurden. Das aus dem Reaktor austretende Gasgemisch wurde getrennt. Ein Anteil von 70 g/h wurde einem Wäscher zugeführt und anschließend abgefakkelt. Der verbliebene Anteil von 18000 g/h des Reaktorausgangsgases wurde bei 60°C in einem Kühler teilkondensiert, der flüssige Anteil mit der Essigsäure in einem Phasentrenner isoliert, und das Restgas zum Reaktoreingang zurückgeführt. Der Säureanteil im Kreisgas wurde so auf 0,5 Vol.-% reduziert. Unter diesen Bedingungen wurde ein Butenumsatz von 99,5 %, ein Butanumsatz von 68 % erreicht. Die Essigsäureausbeute betrug 50 mol-% und die Ameisensäureausbeute 5 mol-%. Die Raum-Zeit-Leistung erreichte 120 g Essigsäure pro Liter Katalysator und Stunde. Die Rohsäurekonzentration betrug 25 Gew.-%.

Beispiel 4:

(Kreisgasfahrweise mit Säureabtrennung nach Abbildung 2)

Es wurde analog Beispiel 3 vorgegangen, wobei der obengenannte Schalenkatalysator eingesetzt wurde, und 700 g/h Wasser, 136 g/h Sauerstoff und 120 g/h n-Butan bei $11 \cdot 10^5$ Pa und 205°C dem Reaktor zugeführt wurden. Das aus dem Reaktor austretende Gasgemisch wurde getrennt. Ein Anteil von 90 g/h wurde einem Wäscher zugeführt und anschließend abgefackelt. Der verbliebene Anteil von 20000 g/h des Reaktorausgangsgases wurde bei 60°C in einem Kühler teilkondensiert, der flüssige Anteil mit der Essigsäure in einem Phasentrenner isoliert, und das Restgas zum Reaktoreingang zurückgeführt. Der Säureanteil im Kreisgas wurde so auf 0,4 Vol.-% reduziert.
Unter diesen Bedingungen wurde ein Butanumsatz von 53 % erreicht. Die Essigsäureausbeute betrug 34 mol-% und die Ameisensäureausbeute 3 mol-%. Die Raum-Zeit-Leistung erreichte 50 g Essigsäure pro Liter Katalysator und Stunde. Die Rohsäurekonzentration betrug 12 Gew.-%.

Beispiel 5:

(Kreisgasfahrweise mit Säureabtrennung nach Abbildung 3)

Ein Reaktor mit einem Reaktionsrohrinnendurchmesser von 25 mm und einer Reaktorlänge von 600 cm, welcher mit einer Heizeinrichtung ausgerüstet war und mit dem obengenannten Katalysator befüllt war, wurde bei einem Druck von $11 \cdot 10^5$ Pa und bei 198°C mit 700 g/h Wasser, 310 g/h Sauerstoff, 120 g/h 1-Buten und 120 g/h n-Butan beschickt.
Die Säureabtrennung erfolgte durch Teilkondensation bei 65°C, wobei der Säureanteil des Reaktorausgangsgases auf 0,65 Vol.-% abgesenkt wurde. 98 Gew.-% des nicht kondensierten Anteils wurden nach der Säureabtrennung in den Reaktor zurückgeführt, sodaß ein Kreisgasfluß von 10000 g/h resultierte. Der Restanteil, 200 g/h des nicht kondensierten Anteils wurde nach der Säureabtrennung der Abgasverbrennung zugeführt.
Unter diesen Bedingungen wurde ein Butenumsatz von 98 % und ein Butanumsatz von 20 % erreicht. Die Essigsäureausbeute betrug 42 mol-%, die Acetaldehydausbeute 0,5 mol-% und die Ameisensäureausbeute 4 mol-%. Die Raum-Zeit-Leistung erreichte 73 g Essigsäure pro Liter Katalysator und Stunde. Die Rohsäurekonzentration betrug 24 Gew.-%.
Die Ergebnisse aus den Beispielen und Vergleichsbeispielen sind in Tabelle 1 zusammengefaßt.

Tabelle 1:

| Beispiel | Butenumsatz (%) | Butanumsatz (%) | Essigsäure (mol-%) | Acetaldehyd (mol-%) | Ameisensäure (mol-%) | Raum/Zeit-Leistung (g/l) | Rohsäure (Gew.-%) |
|---|---|---|---|---|---|---|---|
| Bsp. 1 | 99,5 | | 71 | 0,5 | 5 | 107 | 25 |
| Bsp. 2 | 98 | | 66 | 0,5 | 8,5 | 99 | |
| V.bsp. 1 | 50 | | 34 | 5,0 | 2 | 48 | 5 |
| V.bsp. 2 | 60 | | 36 | 1,2 | 1,2 | 60 | 30 |
| V.bsp. 3 | 46 | | 29 | 3,2 | 2,3 | 50 | 25 |
| Bsp. 3 | 99,5 | 68 | 50 | | 5 | 120 | 25 |
| Bsp. 4 | | 53 | 34 | | 3 | 50 | 12 |
| Bsp. 5 | 98 | 20 | 42 | 0,5 | 4 | 73 | 24 |

**Patentansprüche**

1. Verfahren zur Herstellung von gesättigten Carbonsäuren mit 1 bis 4 C-Atomen durch Gasphasenoxidation bei einer Reaktionstemperatur von 100°C bis 400°C und bei Druck von $1,2 \cdot 10^5$ bis $51 \cdot 10^5$ Pa, in Gegenwart von gesättigten und/oder ungesättigten $C_4$-Kohlenwasserstoffen, einem sauerstoffhaltigen Gas und Wasserdampf in Anwesenheit von zumindest einem Katalysator wobei das Reaktionsausgangsgas in einem Reaktionsgaskreislauf zum Teil zurückgeführt wird, **dadurch gekennzeichnet**, daß der Reaktionsgaskreislauf so ausgeführt wird, daß dem Reaktionsausgangsgas ein Teil der bei der Gasphasenoxidation entstandenen organischen Säuren entzogen werden, sodaß der Säureanteil im zurückgeführten Anteil des Reaktionsausgangsgases auf 0,01 bis 6 Vol.-% reduziert wird .

2. Verfahren nach Anspruch 1, **dadurch gekennzeichnet**, daß der Säureanteil auf 0,2 bis 2 Vol.-% reduziert wird .

3. Verfahren nach Anspruch 1 bis 2, **dadurch gekennzeichnet**, daß bei 60 bis 99,8 Gew.-% des Reaktorausgangsgases der Säureanteil bis zu dem genannten Restsäuregehalt abgetrennt wird, und anschließend dieser Teil des Reaktorausgangsgases wieder in den Reaktor zurückgeführt wird.

4. Verfahren nach Anspruch 1 bis 2, **dadurch gekennzeichnet**, daß dem Reaktorausgangsgas unmittelbar nach dem Austritt aus dem Reaktor der Säureanteil bis zu dem obengenannten Restanteil abgetrennt wird, und das so behandelte Reaktorausgangsgas ganz oder teilweise in den Reaktor zurückgeführt wird.

5. Verfahren nach Anspruch 1 bis 4, **dadurch gekennzeichnet**, daß als $C_4$-Kohlenwasserstoffe n-Butan oder die Buten-Isomere 1-Buten, trans-2-Buten und cis-2-Buten sowie Gemische, die diese Verbindungen in hohen Anteilen enthalten, eingesetzt werden.

6. Verfahren nach Anspruch 5, **dadurch gekennzeichnet**, daß als $C_4$-Kohlenwasserstoffe Rohstoffgemische aus der Petrochemie umfassend "$C_4$-Schnitt" (überwiegender Anteil an Butadien und i-Buten), "Raffinat 1" (überwiegender Anteil an i-Buten und n-Butenen) und "Raffinat 2" (überwiegender Anteil an Butanen, 1-Buten und 2-Butenen) oder Mischungen die solche Kohlenwasserstoffe enthalten, eingesetzt werden.

7. Verfahren nach Anspruch 1 bis 6, **dadurch gekennzeichnet**, daß als sauerstoffhaltiges Gas reiner Sauerstoff, in einer Menge von 1 bis 35 Vol.-% des dem Reaktor zugeführten Reaktoreingangsgases, zugeführt wird.

8. Verfahren nach Anspruch 1 bis 7, **dadurch gekennzeichnet**, daß der Volumenanteil an $C_4$-Kohlenwasserstoff so bemessen wird, daß der Butenanteil 1 bis 5 Vol.-%, allein oder im Gemisch mit weiteren $C_4$-Kohlenwasserstoffen, und/oder der Butananteil 5 bis 80 Vol.-%, allein oder im Gemisch mit weiteren $C_4$-Kohlenwasserstoffen, des dem Reaktoreingang zugeführten Gasgemisches beträgt.

9. Verfahren nach Anspruch 1 bis 8, **dadurch gekennzeichnet**, daß als Katalysator ein Schalenkatalysator eingesetzt wird, bestehend aus einem inerten unporösen Trägerkörper und einer auf die äußere Oberfläche des Trägerkörpers aufgebrachten katalytisch aktiven Mischoxidmasse, welche a) ein oder mehrere Oxide aus der Gruppe Titandioxid, Zirkoniumdioxid, Zinndioxid, Aluminiumoxid und b) 0,1 bis 1,5 Gew.-%, bezogen auf das Gewicht der Komponente a) und pro $m^2$/g spezifischer Oberfläche der Komponente a), Vanadiumpentoxid enthält.

10. Verfahren nach Anspruch 1 bis 9, **dadurch gekennzeichnet**, daß der Anteil des rückgeführten Gasmassenstroms zwischen dem 1-fachen und dem 100-fachen des frisch zugeführten Eduktmassenstroms beträgt.

11. Vorrichtung zur Durchführung des Verfahrens nach Anspruch 1 bis 10 umfassend einen Reaktor (2) mit Zufuhr (1) der mit einem Rohr (5) zur Abtrennung von überschüssigem Gas versehen ist, und mit einem Rohr (3) mit einer Trennvorrichtung (6) zur Teilkondensation bzw. Abtrennung der Säuren verbunden ist, wobei das Rohr (3) von der Trennvorrichtung (6) zur Zufuhr (1) zurückgeführt wird.

12. Vorrichtung zur Durchführung des Verfahrens nach Anspruch 1 bis 10 umfassend einen Reaktor (2) mit Zufuhr (1) wobei der Reaktor (2) über ein Rohr (3) direkt mit einer Trennvorrichtung (6) verbunden ist, mit einem Rohr (5) zur Abführung von überschüssigem Gas aus der Trennvorrichtung (6) ausgestattet ist, wobei mit Rohr (3) von der der Trennvorrichtung (6) zur Zufuhr (1) zurückgeführt wird.

13. Vorrichtung nach Anspruch 11 und 12, **dadurch gekennzeichnet**, daß als Reaktor ein Rohrreaktor eingesetzt wird.

**14.** Vorrichtung nach Anspruch 13, **dadurch gekennzeichnet**, daß als Rohrreaktor ein Rohrbündelreaktor eingesetzt wird.

**15.** Vorrichtung nach Anspruch 11 bis 14, **dadurch gekennzeichnet**, daß der Katalysator im Reaktor als Festbett vorliegt.

**16.** Vorrichtung nach Anspruch 11 bis 14, **dadurch gekennzeichnet**, daß die Trennvorrichtung ein Partialkondensator ist.

**Claims**

**1.** Process for preparing saturated carboxylic acids having from 1 to 4 carbon atoms by gas-phase oxidation at a reaction temperature of from 100°C to 400°C and pressures of from $1.2 \times 10^5$ Pa to $51 \times 10^5$ Pa in the presence of saturated and/or unsaturated $C_4$-hydrocarbons, an oxygen-containing gas and water vapour in the presence of at least one catalyst with the gas leaving the reactor being partly recirculated in a reaction gas circuit, **characterized in that** the reaction gas circuit is configured such that part of the organic acids formed in the gas-phase oxidation is taken from the gas leaving the reactor so that the acid content of the recirculated part of the gas leaving the reactor is from 0.01 to 6.0% by volume.

**2.** Process according to Claim 1, **characterized in that** the acid content is reduced to from 0.2 to 2% by volume.

**3.** Process according to Claim 1 or 2, **characterized in that** the acid content is reduced to the specified residual acid content in from 60 to 99.8% by weight of the reactor outlet gas and this part of the reactor outlet gas is subsequently recirculated to the reactor.

**4.** Process according to Claim 1 or 2, **characterized in that** the acid content of the reactor outlet gas is reduced to the abovementioned residual content immediately after the gas has left the reactor and the reactor outlet gas which has been treated in this way is completely or partially recirculated to the reactor.

**5.** Process according to any of Claims 1 to 4, **characterized in that** $C_4$-hydrocarbons used are n-butane or the butene isomers 1-butene, trans-2-butene and cis-2-butene or mixtures comprising high proportions of these compounds.

**6.** Process according to Claim 5, **characterized in that** $C_4$-hydrocarbons used are feedstock mixtures from petro-chemical processing such as "$C_4$ fraction" (predominantly butadiene and i-butene), "raffinate 1" (predominantly i-butene and n-butenes) and "raffinate 2" (predominantly butanes, 1-butene and 2-butenes) or mixtures comprising such hydrocarbons.

**7.** Process according to any of Claims 1 to 6, **characterized in that**, as oxygen-containing gas, pure oxygen is fed in in an amount of from 1 to 35% by volume of the reactor inlet gas fed to the reactor.

**8.** Process according to any of Claims 1 to 7, **characterized in that** the proportion by volume of $C_4$-hydrocarbon is such that the proportion of butene is from 1 to 5% by volume, either alone or in admixture with further $C_4$-hydrocarbons, and/or the proportion of butane is from 5 to 80% by volume, either alone or in admixture with further $C_4$-hydrocarbons, of the gas mixture fed to the reactor inlet.

**9.** Process according to any of. Claims 1 to 8, **characterized in that** the catalyst used is a coated catalyst comprising an inert nonporous support body and a catalytically active mixed oxide composition comprising a) one or more oxides selected from the group consisting of titanium dioxide, zirconium dioxide, tin dioxide and aluminium oxide and b) from 0.1 to 1.5% by weight, based on the weight of the component a) and per $m^2/g$ of specific surface area of the component a), of vanadium pentoxide applied to the outer surface of the support body.

**10.** Process according to any of Claims 1 to 9, **characterized in that** the mass flow of recirculated gas is from 1 to 100 times the mass flow of fresh starting material fed in.

**11.** Apparatus for carrying out the process according to any of Claims 1 to 10 comprising a reactor (2) which is provided with a feed line (1) and a pipe (5) for separating off excess gas and is connected by means of a pipe (3) to a separation apparatus (6) for partially condensing or separating off the acids, where the pipe (3) returns from the separation apparatus (6) to the feed line (1).

**12.** Apparatus for carrying out the process according to any of Claims 1 to 10 comprising a reactor (2) having a feed line (1), where the reactor (2) is connected by means of a pipe (3) directly to a separation apparatus (6) and is equipped with a pipe (5) for discharging excess gas from the separation apparatus (6), where pipe (3) returns from the separation apparatus (6) to the feed line (1).

**13.** Apparatus according to Claim 11 or 12, **characterized in that** the reactor used is a tube reactor.

**14.** Apparatus according to Claim 13, **characterized in that** the tube reactor used is a multitube reactor.

**15.** Apparatus according to any of Claims 11 to 14, **characterized in that** the catalyst is present in the reactor as a fixed bed.

**16.** Apparatus according to any of Claims 11 to 14, **characterized in that** the separation apparatus is a partial condenser.

**Revendications**

**1.** Procédé pour la préparation d'acides carboxyliques saturés ayant 1 à 4 atomes de carbone par oxydation en phase gazeuse à une température réactionnelle de 100°C jusqu'à 400°C et à une pression de $1{,}2 \cdot 10^5$ jusqu'à $51 \cdot 10^5$ Pa, en présence d'hydrocarbures en $C_4$ saturés et/ou insaturés, d'un gaz contenant de l'oxygène et de la vapeur d'eau en présence d'au moins un catalyseur, le gaz de sortie du réacteur étant recyclé en partie dans un circuit de gaz réactionnel, **caractérisé en ce que** le circuit de gaz réactionnel est réalisé de telle sorte qu'une partie des acides organiques formés au cours de l'oxydation en phase gazeuse soit retirée du gaz de sortie du réacteur afin que la fraction d'acides dans la fraction recyclée du gaz de sortie du réacteur soit réduite à 0,01 jusqu'à 6% en volume.

**2.** Procédé selon la revendication 1, **caractérisé en ce que** la fraction d'acides est réduite à 0,2 jusqu'à 2% en volume.

**3.** Procédé selon les revendications 1 à 2, **caractérisé en ce que**, à 60 jusqu'à 99,8% en poids du gaz de sortie du réacteur, la fraction d'acides est séparée jusqu'à la teneur en acides résiduelle mentionnée, et, ensuite, cette partie du gaz de sortie du réacteur est recyclée dans le réacteur.

**4.** Procédé selon les revendications 1 à 2, **caractérisé en ce que** la fraction d'acides est séparée du gaz de sortie du réacteur directement après la sortie du réacteur jusqu'à la fraction résiduelle précitée et le gaz de sortie du réacteur ainsi traité est recyclé totalement ou partiellement dans le réacteur.

**5.** Procédé selon les revendications 1 à 4, **caractérisé en ce qu'**on utilise comme hydrocarbures en $C_4$ du n-butane ou les isomères de butène, le 1-butène, le trans-2-butène et le cis-2-butène, ainsi que des mélanges qui contiennent ces composés en proportions élevées.

**6.** Procédé selon la revendication 5, **caractérisé en ce qu'**on utilise comme hydrocarbures en $C_4$ des mélanges de matériaux bruts provenant du secteur pétrochimique et comprenant une "fraction en $C_4$" (principalement une fraction de butadiène et de i-butène), un "raffinat 1" (principalement une fraction de i-butène et de n-butènes) et un "raffinat 2" (principalement une fraction de butanes, de 1-butène et de 2-butènes) ou des mélanges qui contiennent ces hydrocarbures.

**7.** Procédé selon les revendications 1 à 6, **caractérisé en ce qu'**on achemine comme gaz contenant de l'oxygène de l'oxygène pur en quantité de 1 à 35% en volume du gaz d'entrée du réacteur acheminé à celui-ci.

**8.** Procédé selon les revendications 1 à 7, **caractérisé en ce que** la fraction volumique en hydrocarbures en $C_4$ est dosée de telle sorte que la fraction de butènes atteigne 1 à 5% en volume, seule ou en mélange avec d'autres hydrocarbures en $C_4$, et/ou que la fraction de butanes atteigne 5 à 80% en volume, seule ou en mélange avec d'autres hydrocarbures en $C_4$, du mélange gazeux acheminé à l'entrée du réacteur.

**9.** Procédé selon les revendications 1 à 8, **caractérisé en ce qu'**on utilise comme catalyseur un catalyseur enrobé constitué d'un corps de support inerte non poreux et d'une masse d'oxydes mixtes à activité catalytique appliquée sur la surface extérieure du corps de support et qui contient a) un ou plusieurs oxydes du groupe du dioxyde de titane, du dioxyde de zirconium, du dioxyde d'étain et du dioxyde d'aluminium, et b) 0,1 à 1,5% en poids de pentoxyde de vanadium par rapport au poids du composant a) et par $m^2/g$ de surface spécifique du composant a).

10. Procédé selon la revendication 1 à 9, **caractérisé en ce que** la fraction du courant massique de gaz recyclé atteint entre 1 fois et 100 fois le courant massique effluent fraîchement acheminé.

11. Dispositif pour réaliser le procédé selon les revendications 1 à 10, comprenant un réacteur (2) avec une alimentation (1), qui est pourvu d'un tube (5) pour la séparation du gaz excédentaire et est raccordé par un tube (3) à un dispositif de séparation pour la condensation partielle ou selon le cas la séparation des acides, le tube (3) étant renvoyé du dispositif de séparation (6) à l'alimentation (1).

12. Dispositif pour réaliser le procédé selon les revendications 1 à 10, comprenant un réacteur (2) avec une alimentation (1), dans lequel le réacteur (2) est raccordé via un tube (3) directement à un dispositif de séparation (6) et est équipé d'un tube (5) pour l'évacuation de gaz excédentaire du dispositif de séparation (6), le tube (3) étant renvoyé du dispositif de séparation (6) à l'alimentation (1).

13. Dispositif selon les revendications 11 et 12, **caractérisé en ce qu'**on utilise comme réacteur un réacteur tubulaire.

14. Dispositif selon la revendication 13, **caractérisé en ce qu'**on utilise comme réacteur tubulaire un réacteur en faisceau de tubes.

15. Dispositif selon les revendications 11 à 14, **caractérisé en ce que** le catalyseur se présente sous la forme d'un lit fixe dans le réacteur.

16. Dispositif selon les revendications 11 à 14, **caractérisé en ce que** le dispositif de séparation est un condenseur partiel.

Figur 1

Figur 2

Figur 3